# EUROPÄISCHE PATENTSCHRIFT

(11) **EP 2 750 588 B1**
(45) Veröffentlichungstag und Bekanntmachung des Hinweises auf die Patenterteilung: **21.03.2018**
(21) Anmeldenummer: 12745408.0
(22) Anmeldetag: 04.08.2012
(51) Int. Cl.: A61B 5/024, A61B 5/00

(54) **VERFAHREN ZUR ZUSTANDSERKENNUNG EINES FAHRZEUGFÜHRERS DURCH ERFASSUNG EINER PLETHYSMOGRAPHISCHEN GRÖSSE**
METHOD FOR IDENTIFYING THE STATE OF A VEHICLE DRIVER BY MEASURING A PLETHYSMOGRAPHIC VARIABLE
PROCÉDÉ POUR LA DÉTECTION DE L'ÉTAT D'UN CONDUCTEUR DE VÉHICULE OPÉRANT PAR SAISIE D'UNE GRANDEUR PLÉTHYSMOGRAPHIQUE

(30) Priorität: 03.09.2011 DE 102011112344
(43) Veröffentlichungstag der Anmeldung: 09.07.2014
(73) Patentinhaber: Volkswagen Aktiengesellschaft, 38440 Wolfsburg (DE)
(72) Erfinder: TO, Thanh-Binh, 39179 Barleben (DE)
(86) Internationale Anmeldenummer: PCT/EP2012/003334
(87) Internationale Veröffentlichungsnummer: WO 2013/029738

(56) Entgegenhaltungen:
- WO-A1-2011/042839
- MING-ZHER POH ET AL: "Non-contact, automated cardiac pulse measurements using video imaging and blind source separation", OPTICS EXPRESS, Bd. 18, Nr. 10, 10. Mai 2010 (2010-05-10), Seite 10762, XP055016649, ISSN: 1094-4087, DOI: 10.1364/OE.18.010762
- WIM VERKRUYSSE1 ET AL: "Remote plethysmographic imaging using ambient light", OPTICS EXPRESS, OSA (OPTICAL SOCIETY OF AMERICA), WASHINGTON DC, (US), Bd. 16, Nr. 26, 22. Dezember 2008 (2008-12-22), Seiten 21434-21445, XP007913060, ISSN: 1094-4087

## Beschreibung

Die Erfindung betrifft ein Verfahren zur Zustandserkennung eines Fahrzeugführers eines Kraftfahrzeugs durch Erzeugung einer eines den physiologischen Zustand des Fahrzeugführers anzeigenden plethysmographischen Größe gemäß Oberbegriff des Patentanspruchs 1 und des Patentanspruchs 2. Optische plethysmographische Verfahren, sogenannte photo-plethysmogra-phische Verfahren sind als nicht-invasive Methoden zur Erfassung von physiologischen Parametern bzw. Vitalparametern, wie Pulsrate, Pulsratenvariabilität oder arteriellen Sauerstoffsättigung im Blut einer Person bekannt. Bei diesen Verfahren wird rotes oder infrarotes Licht in das Körpergewebe eingestrahlt und über die Messung der Lichtabsorption bzw. Lichtremission ein Volumenpulssignal erzeugt, das die während des Herzschlags veränderliche Blutmenge in dem Gefäßsystem des erfassten Körpergewebes anzeigt. Die für dieses Verfahren eingesetzten Geräte, sogenannte Pulsoximeter werden entweder an der Fingerspitze einer Person oder an deren Ohrläppchen eingesetzt.

So ist aus der gattungsbildenden DE 10 2008 056 250 A1 ein Verfahren zum Erfassen zumindest eines Vitalparameters, insbesondere der Pulsinformation einer Person in einem Kraftfahrzeug beschrieben, bei dem mittels einer optoelektronischen Sensoranordnung der Vitalparameter mittels Lichtremission erfasst wird, wobei die optoelektronische Sensoranordnung zumindest eine erste Lichtquelle und ein lichtempfindliches Element aufweist und die erste Lichtquelle und das lichtempfindliche Element in einem Fingerbett eines Bedienelements, insbesondere eines Schaltknaufs oder des Lenkrads des Kraftfahrzeugs angeordnet sind. Das Fingerbett nimmt die Fingerkuppe der Person in einem Sensorbereich des Fingerbetts, in dem die erste Lichtquelle und das lichtempfindliche Element angeordnet sind, bündig auf.

Dieses bekannte Verfahren kann im Rahmen eines Fahrerassistenzsystems eingesetzt werden, indem wenigstens ein Vitalparameter mit einem gegebenen Schwellwert verglichen wird, um eine Warnmeldung, ein Alarm oder einen Notruf auszugeben bzw. auszulösen, wenn der Wert des Vitalparameters diesen Schwellwert über- oder unterschreitet. Mit einem solchen Assistenzsystem können kardiologische Risikofaktoren, eine chronische Lungenerkrankung der Person überwacht oder Müdigkeit und/oder Stress der Person erkannt werden.

Der Nachteil dieses bekannten Verfahren liegt darin, dass der Fahrerzustand nicht laufend überwacht bzw. detektiert werden kann, da dieser nur dann erfasst werden kann, wenn der Fahrer gerade ein mit dem Fingerbett ausgestattetes Bedienelement, wie bspw. den Schaltknauf des Fahrzeugs betätigt. Es kann nicht davon ausgegangen werden, dass ein Fahrzeugführer seine Hand ständig an einem solchen Bedienelement belässt. Selbst wenn dieses Bedienelement das Lenkrad darstellt, kann ein solches Fingerbett nur an wenigen Stellen des Lenkradkranzes angeordnet werden und daher nicht davon ausgegangen werden, dass die Hände des Fahrers ständig dort liegen, an denen die Fingerbetten sich befinden. Neben dem Nachteil einer aufwendigen konstruktiven Realisierung solcher Fingerbetten am Lenkradkranz, würde auch der optische Eindruck durch solche auf dem Lenkradkranz angeordneten Fingerbetten nachhaltig beeinträchtig.

Ferner ist ein Verfahren zur Charakterisierung des Zustandes des Fahrers eines Kraftfahrzeuges aus der DE 101 26 224 A1 bekannt, bei dem ein Fahrer-Monitoring durchgeführt wird und als Schlüsselparameter physiologische Zustandsgrößen des Fahrers als Kenngrößen erfasst werden und anschließend ein Vergleich der erfassten Daten mit gespeicherten Daten und/oder eine direkte Auswertung der erfassten Daten durchgeführt wird, um in Abhängigkeit vom ermittelten Ergebnis auf Steuerungseinheiten des Kraftfahrzeugs einzuwirken, die geeignete Maßnahmen zur Einhaltung eines sicheren Zustands des Fahrers und/oder des Kraftfahrzeugs einleiten.

Als physiologische Zustandsgrößen des Fahrers werden bei diesem bekannten Verfahren Gehirnströme mittels eines Elektroenzephalogramms (EEG) erfasst, wobei das Elektroenzephalogramm mit zumindest zwei Elektroden im Kopfbereich des Fahrers aufgenommen und aus dem Übergang von beta- zu alpha-Wellen auf den Wachheitszustand des Fahrers geschlossen wird. Weiterhin werden als physiologische Zustandsgrößen der Herzzustand, z. Bsp. der Herzschlag- bzw. die Pulsfrequenz und/oder die Herzfrequenz mittels eines Elektrokardiogramms (EKG) und der Blutdruck mittels Blutdruckmessung erfasst.

Zur Erfassung dieser Zustandsgrößen des Fahrers sind geeignete Sensoren vorgesehen, welche die Bewegungsfreiheit des überwachten Fahrers in der Fahrposition möglichst wenig oder gar nicht beeinträchtigen sollen. Zusätzlich wird auch ein optischer Sensor, wie eine Kamera vorgeschlagen, der auf das Gesicht des Fahrers ausgerichtet ist, so dass die Gesichtsbewegungen und insbesondere der Augenlidschluss bzw. die Frequenz, mit welcher die Augenlider des Fahrers geschlossen werden, als weitere Zustandsgrößen des Fahrers erfasst und ausgewertet werden können.

Bei diesem bekannten Verfahren gemäß der DE 101 26 224 A1 wird die Herztätigkeit, also bspw. der Puls bzw. der Herzschlag des Fahrers mittels Sensoren erfasst, die einen Kontakt mit dem Körper des Fahrers erfordern, also Elektroden und daher den Fahrer in seiner Bewegungsfreiheit behindern.

Weiterhin ist aus dem gattungsbildenden Artikel "Non-contact, automated cardiac pulse measurements using video imaging and blind source separation"; Poh, M., McDuff, D. J., Picard, R.W., Optics Express, Vol. 18, Issue 10, pp. 10762-10774 (2010) ein Verfahren zur berührungslosen Herzpulsmessung beschrieben, bei dem das oben beschriebene Photoplethysmographie-Verfahren verwendet wird, jedoch ohne eine zusätzliche rote oder infrarote Lichtquelle einzusetzen. Von einer üblichen Videokamera wird der Kopf einer Person erfasst und aus den Bilddaten das Gesicht dieser Person extrahiert; das von dem Gesicht reflektierte Tageslicht in seine roten, grünen und blauen Anteile zu zerlegen, um aus den Daten dieser Farbkanäle mittels eines Blind-Quellentrennungsprozess unabhängige Signalquellen, nämlich die physiologischen Signale zu extrahieren, wobei die separierten Signalquellen die plethysmographischen Signale darstellen.

Schließlich ist aus dem Artikel "Remote plethysmographic imaging using ambient light"; Verkruysse, W., Svaasand, L. O., Nelson, J. S.; Optics Express, Vol. 16, Issue 26, pp. 21434-21445 (2008) bekannt, dass die Grün-Anteile der bei Umgebungslicht mittels einer Videokamera aufgenommenen Bilddaten eines Gesichtes die stärksten plethysmographischen Signale im Vergleich zu den Rot- und Blau-Anteilen des reflektierten Tageslichtes aufweisen.

Diese beiden zuletzt beschriebenen Verfahren zur Erzeugung von plethysmographischen Signalen bei Umgebungslicht verwenden für dieses "Umgebungslicht-PhotoPlethysmographie"-Verfahren eine Farb-Videokamera, welche für den Einsatz in einem Kraftfahrzeug hohe Kosten verursacht. Zusätzlich ist die Bearbeitung und Auswertung der Rohbilddaten, also der hierfür erforderliche mathematische Algorithmus sehr aufwendig und erfordert deshalb eine hohe Rechenkapazität, wodurch hierfür ebenso hohe Kosten entstehen. Weiterhin nachteilig ist bei einer Verwendung einer Farbkamera deren geringeren Ortsauflösung gegenüber einer monochromen Kamera

Die zwei-teilige Form der Ansprüche 1 und 2 basiert auf dem folgenden Dokument: MING-ZHER POH ET AL: "Non-contact, automated cardiac pulse measurements using video imaging and blind source separation", OPTICS EXPRESS, Bd. 18, Nr. 10, 10. Mai 2010 (2010-05-10), Seite 10762. Aufgabe der Erfindung ist es, ein Verfahren zur Zustandserkennung eines Fahrzeugführers durch Erfassung einer plethysmographischen Größe bereitzustellen, welches eine schnelle und einfache Zustandserkennung eines Fahrzeugführers eines Kraftfahrzeugs erlaubt, mit dem aber dennoch robuste Ergebnisse erzielbar sind und schließlich auch kostengünstig realisierbar ist.

Ferner ist es Aufgabe der Erfindung eine Vorrichtung zur Durchführung des erfindungsgemäßen Verfahrens anzugeben, welches kostengünstig realisiert werden kann.

Die erstgenannte Aufgabe wird gelöst durch ein Verfahren mit den Merkmalen des Patentanspruchs 1, sowie durch ein Verfahren mit den Merkmalen des Patentanspruchs 2.

Ein solches Verfahren zur Zustandserkennung eines Fahrzeugführers eines Kraftfahrzeugs durch Erzeugung eines den physiologischen Zustand des Fahrzeugführers anzeigende plethysmographischen Größe, vorzugsweise die Herzschlagrate, mittels eines im Fahrzeug angeordneten Sensors, zeichnet sich gemäß der erstgenannten Lösung erfindungsgemäß dadurch aus, dass
- der Sensor als monochromer Bildsensor zur Bildaufnahme des Kopfes des Fahrzeugführers ausgebildet ist,
- ein strukturierter Grünfilter vorgesehen ist, der grüne und transparente Filterpixel aufweist,
- aus der Bildaufnahme des Kopfes der Gesichtsbereich des Fahrzeugführers detektiert wird,
- von mehreren Einzelbildern des Kopfes des Fahrzeugführers die Grün-Anteile des Gesichtsbereiches erfasst und jeweils über eine vorgegebene Zeitdauer als Bilddaten gespeichert werden, und
- aus den gespeicherten Bilddaten mittels eines mathematischen Algorithmus die plethysmographische Größe bestimmt wird, wobei
- der Grünfilter wenigstens eine 2x2-Matrix von Filterpixeln umfasst, die abwechselnd grüne Filterpixel und transparente Filterpixel aufweist.

Die zweitgenannte Lösung zeichnet sich dadurch aus, dass der Sensor als Bildsensor mit in Spalten und Reihen angeordneten lichtempfindlichen Elementen ausgebildet ist, wobei in einem Muster lichtempfindliche Elemente einen Grünfilter aufweisen, aus der Bildaufnahme des Kopfes der Gesichtsbereich des Fahrzeugführers detektiert wird,
- von mehreren Einzelbildern des Kopfes des Fahrzeugführers die Grün-Anteile des Gesichtsbereiches erfasst und jeweils über eine vorgegebene Zeitdauer als Bilddaten gespeichert werden, und
- aus den gespeicherten Bilddaten mittels eines mathematischen Algorithmus die plethysmographische Größe bestimmt wird, wobei
- der Bildsensor wenigstens eine 2x2-Matrix von lichtempfindlichen Elementen umfasst, die abwechselnd einen Grünfilter und keinen Filter aufweisen

Die zweitgenannte Lösung unterscheidet sich von der erstgenannten lediglich dadurch, dass anstelle des separaten Farbfilters ein Bildsensor (Image-chip) vorgesehen ist, bei dem in dessen Herstellungsprozess der grüne Farbfilter auf das Halbleitermaterial aufgebracht wird.

Bei diesen beiden Lösungen des erfindungsgemäßen Verfahrens gibt es Bildpunkte, die von transparenten Filterpixeln bzw. Sensorpixeln ohne Filter geliefert werden und die Intensität des detektierten Lichts als Grauwert angeben, sowie Bildpunkte, die von grünen Filterpixeln bzw. grünen Sensorpixeln erzeugt werden und die die Intensität der Farbe Grün angeben. Dies hat zur Folge, dass die für monochrome Bildsensoren typische hohe Auflösung nahezu erhalten bleibt.

Durch die Verwendung eines monochromen Bildsensors mit strukturiertem Grünfilter mit grünen und transparenten Filterpixeln bzw. eines Bildsensors mit grünen Sensorpixeln und Sensorpixeln ohne Filter werden gegenüber der Verwendung einer Farbkamera robuste und zuverlässige Messergebnisse erzielt, so dass dadurch ohne hohen Softwareaufwand eine hohe Erkennungssicherheit eines plethysmographischen Signals in den Bilddaten erreichbar ist. Durch die Verwendung eines monochromen Farbsignals der Farbe Grün zur Erzeugung eines plethysmographischen Signals ist dieses erfindungsgemäße Verfahren auch schneller als die aus dem Stand der Technik bekannten Verfahren.

Dadurch, dass bei der Bildaufnahme des Kopfes des Fahrzeugführers auch Bereiche ohne Farbfilterung detektiert werden, kann das Gesicht auch bei Dunkelheit so gut detektiert werden, dass eine zusätzliche Beleuchtung mit Infrarot-Licht verzichtbar ist.

Schließlich ist die Verwendung eines solchen monochromen Bildsensors mit strukturiertem Grünfilter bzw. eines Bildsensors mit grünen Sensorpixeln und Sensorpixeln ohne Filter wesentlich kostengünstiger als der Einsatz eines Farb-Bildsensors.

Dadurch dass die Lage der grünen Filterpixel bzw. der grünen Sensorpixel bekannt ist, lässt sich die Extraktion der Grün-Anteile dieser grünen Filterpixel bzw. grünen Sensorpixel einfach durchführen.

In einer Ausgestaltung der Erfindung gemäß der ersten und zweiten Lösung ist es besonders vorteilhaft, wenn zur Bestimmung des Grün-Anteils eines Einzelbildes des Kopfes des Fahrzeugführers das HSV-Farbsystem (Hue Saturation Value) verwendet wird. Durch die Verwendung dieses HSV-Systems zur Bilddaten- bzw. Signalverarbeitung anstelle des weit verbreiteten RGB-Farbsystems (Rot - Gelb - Blau) und die alleinige Auswertung des Grün-Anteils eines Einzelbildes wird die Erkennungsrate eines plethysmographischen Signals wesentlich erhöht. Da vorzugsweise als Bilddaten der Grün-Farbton eines Einzelbildes und dessen Sättigungswerte bei der Auswertung der Bilddaten des Gesichtsfeldes bestimmt werden, wird die Verarbeitungsgeschwindigkeit erhöht.

Weiterhin ist gemäß einer vorteilhaften Weiterbildung der Erfindung gemäß der erstgenannten und zweitgenannten Lösung vorgesehen, die Mittelwerte der Grün-Werte eines Einzelbildes über mehrere Einzelbilder zu speichern, wodurch auch unter unterschiedlichen oder schnell wechselnden Lichtbedingungen, wie dies im Betrieb eines Kraftfahrzeugs auftreten kann, das erfindungsgemäße Verfahren zuverlässige und robuste Ergebnisse liefert.

Besonders vorteilhaft ist es gemäß einer weiteren Ausgestaltung der Erfindung, wenn die gespeicherten Bilddaten mittels Fourier Transformation, insbesondere mittels einer schnellen Fourier Transformation (Fast Fourier Transformation, FFT) in den Frequenzbereich transformiert werden und aus dessen Energie-Spektrum (Spektrogramm) die plethysmographische Größe bestimmt wird. Mit einer solchen Fourier Transformation wird eine schnelle Bild- bzw. Signalauswertung erzielt.

Besonders kostengünstig ist es gemäß einer Weiterbildung der Erfindung, wenn zur Bildaufnahme des Kopfes des Fahrzeugführers eine monochrome Videokamera mit einem separaten Grün-Filter verwendet wird. Damit kann ein kostengünstiger CCD- oder ein CMOS-Bildsensor eingesetzt werden, wobei ein externes handelsübliches Grün-Filter ebenso nur geringe Kosten verursacht.

Das erfindungsgemäße Verfahren eignet sich besonders dafür, aus dem plethysmographischen Signal die Herzschlagrate und/oder Atemfrequenz und/oder Herzfrequenzvariabilität und/oder die Sauerstoffsättigung des arteriellen Bluts abzuleiten.

Schließlich kann gemäß einer letzten Weiterbildung der Erfindung das erfindungsgemäße Verfahren in seiner Zuverlässigkeit und Robustheit weiter verbessert werden, wenn das strukturierte Grünfilter nur grüne Filterpixel bzw. der Bildsensor nur lichtempfindliche Elemente mit Grünfilter aufweist und der Kopf des Fahrzeugführers mit einer roten oder infraroten Lichtquelle, insbesondere einer infrarotnahen Lichtquelle beleuchtet wird, wobei hierfür insbesondere der Frequenzbereich zwischen 700 und 900 nm geeignet ist. Die Ursache liegt darin, dass die Absorption des das Gefäßsystem füllenden venösen und pulsierenden arteriellen Blutes in diesem Frequenzbereich besonders hoch ist und daher ein deutliches blutfüllungsabhängiges Signal erzeugt werden kann. Ferner ist zur Detektion des Gesichtsfeldes (ROI) bei Dunkelheit besser, zur Ausleuchtung des Kopfes nahes Infrarotlicht einzusetzen.

Die Erfindung wird nachfolgend anhand von Ausführungsbeispielen unter Bezugnahme auf die beigefügten Figuren ausführlich beschrieben. Es zeigen:
- Figur 1: eine schematische Darstellung einer Vorrichtung zur Zustandserkennung eines Fahrzeugführers als Blockdiagramm zur Durchführung des erfindungsgemäßen Verfahrens,
- Figur 2: eine schematische Darstellung eines monochromen Bildsensors mit Zeilen und Spalten,
- Figur 3: eine schematische Darstellung eines Grünfilters mit grünen und transparenten Filterpixeln, und
- Figur 4: eine schematische Darstellung eines Bildsensors mit in Zeilen und Spalten angeordneten grünen Sensorpixeln und Sensorpixeln ohne Filter.

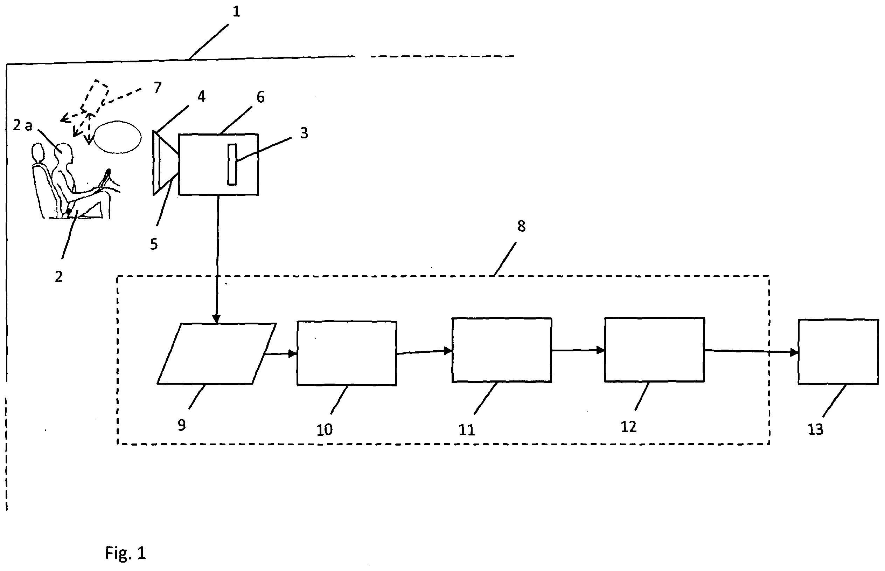

Die Vorrichtung gemäß Figur 1 zur Zustandserkennung eines Fahrzeugführers 2 in einem Kraftfahrzeug 1 umfasst eine monochrome Videokamera 6 mit einem externen Grünfilter 4, eine Auswerteeinheit 8, bspw. als Mikroprozessor ausgebildet und einem dieser Auswerteinheit 8 nachgeschalteten Fahrerassistenzsystem 13. Die Auswerteeinheit 8 besteht aus mehreren Funktionseinheiten 9, 10, 11 und 12.

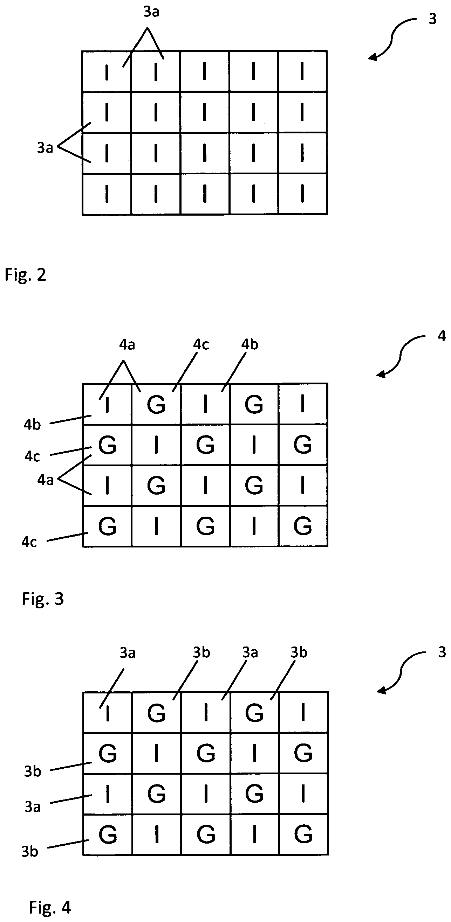

Die Videokamera 6 umfasst eine Optik bzw. ein Objektiv 5 und einen Image-Chip als Bildsensor 3, der als CCD- oder CMOS-Chip ausgebildet ist und gemäß Figur 2 aus in Zeilen und Spalten angeordneten Sensorelementen 3a besteht, die als Intensitätspixeln Grauwerte liefern. Jedes dieser Intensitätspixel 3a liefert eine als "I" bezeichnete Intensitäts-Information.

Der Grünfilter 4 dieser Videokamera 6 ist als externer Filter vor der Optik bzw. dem Objektiv 5 der Videokamera 6 angeordnet, so dass als Videokamera 6 eine Standard-Monochrom-Kamera eingesetzt werden kann.

Der Grünfilter 4 ist mit seinen Filterpixeln 4a in Zeilen und Spalten aufgebaut und derart strukturiert, dass ein transparenter Filterpixel 4b mit einem grünen Filterpixel 4c beginnend oben links fortlaufend abwechselt. Die transparenten Filterpixel 4b lassen das Licht ungefiltert auf den zugeordneten Sensorpixel 3a durch, während die grünen Filterpixel 4c nur die grüne Farbe auf den zugeordneten Sensorpixel 3a durchlassen.

Ein solches Grünfilter 4 kann anstelle einer Anordnung vor der Optik bzw. dem Objektiv 5 der Videokamera 6 auch innerhalb der Kamera vor dem Image-Chip 3 vorgesehen werden.

Alternativ kann auch eine Videokamera 6 verwendet werden, die einen in Figur 4 dargestellten Bildsensor 3 aufweist, dessen lichtempfindliche Sensorelemente ebenfalls in Zeilen und Spalten angeordnet sind. Dieser Bildsensor weist in einer regelmäßigen Struktur angeordnete Sensorelemente 3b mit einem grünen Filterelement auf, so dass sich Sensorelemente 3a ohne Filter mit Sensorelementen 3b mit einem grünen Filterelement beginnend oben links abwechseln. Die Sensorelemente 3a liefern daher einen dem Umgebungslicht entsprechenden Grau-Wert, also einen Intensitätswert, während die Sensorelemente 3b einen Intensitätswert der Farbe Grün liefern.

Die Videokamera 6 erzeugt von dem Kopf 2a des Fahrzeugführers 2 aus Einzelbildern (frames) bestehende Bilddaten, die als Roh-Bilddaten in der Funktionseinheit "Gesichtserkennung" 9 zwischengespeichert werden, um mit diesen Roh-Bilddaten mittels einer geeigneten Methode eine biometrische Gesichtserkennung durchzuführen und den Gesichtsbereich des Kopfes 2a des Fahrzeugführers 2 als ROI (Region-Of-Interest) der nachfolgenden Funktionseinheit "Extrahieren Grün-Anteil" 10 zuzuführen, mit der der Grün-Anteil aus dem ROI extrahiert wird.

Zur Gesichtserkennung werden alle Pixel eines Einzelbildes (frame) verwendet, also solche die ungefilterte Intensitätswerte als auch Intensitätswerte der Farbe Grün liefern. Die ungefilterten Intensitätswerte liefern einen dem Umgebungslicht entsprechenden Grau-Wert.

Der Grün-Anteil der ROI wird dagegen nur aus den Pixeln extrahiert, die einen Intensitätswert der Farbe Grün detektiert haben.

Zur Bestimmung des Grün-Anteils werden zunächst die Grünwerte aus jedem Einzelbild (frame) extrahiert und der Mittelwert dieser Grün-Werte bestimmt. Da die Struktur der die Farbe Grün anzeigender Sensorelemente des Image-Chips 3, also deren Ort bekannt ist, kann die Extraktion des Grün-Anteils softwaremäßig einfach durchgeführt werden.

Für diese Farbraumtransformation wird das HSV-Farbsystem verwendet, wobei nur der Farbton Grün (Hue-Anteil) und die Sättigung (Saturation-Anteil) berücksichtigt werden, also ohne die Helligkeit (Value-Anteil), deren Wert für alle Bildpixel des ROI, also des Gesichtsbereiches auf einen gleichen Wert, also bspw. auf einen Default-Wert gesetzt wird.

Durch die Verwendung dieses HSV-Farbsystems wird erreicht, dass trotz sich ständig ändernden Lichtbedingungen während des Fahrens eine hohe und zuverlässige Funktionssicherheit hinsichtlich der Erkennung eines plethysmographischen Signals sichergestellt wird. Da auch das Umgebungslicht ebenfalls detektiert wird, ist unter allen Lichtbedingungen eine sichere Detektion des Gesichts möglich.

Die für jedes Einzelbild (Frame) ermittelten Durchschnittswerte der zeitdiskreten H- und S-Werte im HSV-Farbraum werden für eine bestimmte Zeitdauer, bspw. 30 s in dem Zwischenspeicher 11, bspw. als Histogramm gespeichert.

Eine alternative Farbraumtransformation besteht darin, dass direkt die Sensorsignale der Sensorelemente des Bildsensors 3, die einen Intensitätswert der Farbe Grün liefern, als Bilddaten verwendet werden. Diese Bilddaten enthalten nur Graustufen und repräsentieren die Helligkeitsunterschiede des ROI, die zur Bildauswertung verwendet werden. Auch bei diesem alternativen Verfahren wird für jedes Einzelbild (Frame) ein Intensitäts-Mittelwert ermittelt und für eine bestimmte Zeitdauer, bspw. 30 s in dem Zwischenspeicher 11 gespeichert.

Eine weitere Ausführung der Videokamera 6 gemäß Figur 1 besteht aus einem Filter 4, der ein vollständiges Grünfilter darstellt, dessen Filterpixel 4a alle aus einem grünen Filterpixel 4c bestehen. Damit stehen nur die Intensitätswerte der Farbe Grün als Bilddaten zur Auswertung zur Verfügung.

Solche Bilddaten werden auch mit einer Videokamera 6 erzeugt, bei der der Bildsensor 3 eine Struktur von Sensorelementen 3b aufweist, die alle mit einem Grünfilter ausgebildet sind.

Damit mit solchen Bilddaten die Gesichtserkennung mittels der Funktionseinheit "Gesichtserkennung" 9 zuverlässig durchführbar ist, ist es vorteilhaft, die Vorrichtung nach Figur 1 mit einer Nah-Infrarot-Lichtquelle 7 auszustatten, die den Kopf des Fahrzeugführers 2a mit Infrarotlicht beleuchtet.

Die derart erzeugten Bilddaten werden gemäß dem oben beschriebenen Verfahren ausgewertet und die für jedes Einzelbild (Frame) ermittelten Durchschnittswerte der zeitdiskreten H- und S-Werte im HSV-Farbraum bzw. der mittels der alternativen Farbtransformation ermittelten Intensitäts-Mittelwerte für jedes Einzelbild (frame) in dem Zwischenspeicher 11 für eine bestimmte Zeitdauer gespeichert.

Mit der Funktionseinheit "FFT" 12 werden diese zeitdiskreten H- und S-Werte bzw. die zeitdiskreten Intensitäts-Mittelwerte mittels der Fast Fourier Transformation (FFT) in den Frequenz-Bereich transformiert. Mittels einer geeigneten mathematischen Methode wird im Energiespektrum (Spektrogramm) nach einem dem Frequenzbereich von 0,65 Hz bis 4 Hz entsprechenden Peak als plethysmographischen Signal gesucht, da dieser Frequenzbereich einem Herzschlag von 39 bis 240 Schlägen pro Minute entspricht. Anhand eines solchen Peaks in dem genannten Frequenzbereich wird die aktuelle Herzschlagrate für die bestimmte Zeitdauer, also bspw. 30 s berechnet. Anschließend wird für die darauffolgende Zeitdauer die Herzschlagrate bestimmt.

Mit einer solchen Folge von Herzschlagraten können weitere Auswertungen vorgenommen werden, um bspw. die Herzfrequenzvariabilität zu bestimmen. Auch können die erhaltenen Herzschlagraten mittels eines geeigneten Kalman-Filters fusioniert werden, um die Genauigkeit der Herzschlagrate oder der Herzfrequenzvariabilität zu erhöhen. Anstelle eines Kalman-Filters können auch probabalistische Methoden oder eine beliebige Fusionsmethode verwendet werden.

Entsprechend dem beschriebenen Verfahren kann aus dem Spektrogramm auch die Atemfrequenz oder die Sauerstoffsättigung des arteriellen Bluts des Fahrzeugführers bestimmt werden.

Die oben erwähnte Nah-Infrarot-Lichtquelle 7 gemäß Figur 1 kann auch generell zur verbesserten Erkennung eines plethysmographisches Signals eingesetzt werden. Da es bekannt ist, dass im Frequenzbereich zwischen 700 und 900 nm die Absorption des das Gefäßsystem füllenden venösen und pulsierenden arteriellen Blutes besonders hoch ist, führt die Beleuchtung des Kopfes 2a des Fahrzeugführers 2 mit Infrarotlicht zu einem deutlicheren blutfüllungsabhängigen Signal.

Die ausgewerteten plethysmographischen Signale, also bspw. Herzschlagrate und/oder Atemfrequenz werden einem Fahrerassistenzsystem 13 zugeführt, so dass bspw. bei einem bestimmten Wert dieser plethysmographischen Signale ein Nothalt durchgeführt wird, d. h. dass das Fahrzeug automatisch bis zum Stillstand abgebremst wird. Gleichzeitig kann in einem solchen Notfall über eine Sendeeinrichtung im Fahrzeug ein entsprechender Notruf an eine Rettungsleitstelle abgesetzt werden, wobei zusätzlich auch Positionsdaten des Fahrzeugs, ermittelte Gesundheitsdaten (Herzschläge, Atmung etc.), sowie Gesichtsvideos etc. übermittelbar wären.

### Bezugszeichenliste

- 1: Kraftfahrzeug
- 2: Fahrzeugführer
- 2a: Kopf des Fahrzeugführers 2
- 3: Bildsensor
- 4: Grünfilter
- 5: Optik, Objektiv der Videokamera
- 6: Videokamera
- 7: infrarote oder rote Lichtquelle
- 8: Auswerteeinheit
- 9: Funktionseinheit "Gesichtserkennung"
- 10: Funktionseinheit "Extrahieren Grün-Anteil"
- 11: Zwischenspeicher
- 12: Funktionseinheit "FFT"
- 13: Fahrerassistenzsystem

## Patentansprüche

1. Verfahren zur Zustandserkennung eines Fahrzeugführers (2) eines Kraftfahrzeugs (1) durch Erzeugung einer den physiologischen Zustand des Fahrzeugführers (2) anzeigenden plethysmographischen Größe, vorzugsweise der Herzschlagrate mittels eines im Fahrzeug (1) angeordneten Sensors (3), wobei
- der Sensor als monochromer Bildsensor (3) zur Bildaufnahme des Kopfes (2a) des Fahrzeugführers (2) ausgebildet ist,
- ein strukturierter Grünfilter (4, 4a) vorgesehen ist, der grüne und transparente Filterpixel (4b, 4c) aufweist,
- aus der Bildaufnahme des Kopfes (2a) der Gesichtsbereich des Fahrzeugführers (2) detektiert wird,
- von mehreren Einzelbildern des Kopfes (2a) des Fahrzeugführers (2) die Grün-Anteile des Gesichtsbereiches erfasst und jeweils über eine vorgegebene Zeitdauer als Bilddaten gespeichert werden, und
- aus den gespeicherten Bilddaten mittels eines mathematischen Algorithmus die plethysmographische Größe bestimmt wird,
**dadurch gekennzeichnet, dass** der Grünfilter (4, 4a) wenigstens eine 2x2-Matrix von Filterpixeln (4b, 4c) umfasst, die abwechselnd grüne Filterpixel (4c) und transparente Filterpixel (4b) aufweist.

2. Verfahren zur Zustandserkennung eines Fahrzeugführers (2) eines Kraftfahrzeugs (1) zur Erzeugung einer den physiologischen Zustand des Fahrzeugführers (2) anzeigenden plethysmographischen Größe, vorzugsweise der Herzschlagrate mittels eines im Fahrzeug (1) angeordneten Sensors (3), wobei
- der Sensor als Bildsensor (3) zur Bildaufnahme des Kopfes (2a) des Fahrzeugführers (2) und mit in Spalten und Reihen angeordneten lichtempfindlichen Sensorelementen (3a, 3b) ausgebildet ist, wobei in einem Muster lichtempfindliche Sensorelemente (3b) einen Grünfilter aufweisen,
- aus der Bildaufnahme des Kopfes (2a) der Gesichtsbereich des Fahrzeugführers (2) detektiert wird,
- von mehreren Einzelbildern des Kopfes (2a) des Fahrzeugführers (2) die Grün-Anteile des Gesichtsbereiches erfasst und jeweils über eine vorgegebene Zeitdauer als Bilddaten gespeichert werden, und
- aus den gespeicherten Bilddaten mittels eines mathematischen Algorithmus die plethysmographische Größe bestimmt wird,
**dadurch gekennzeichnet, dass** der Bildsensor (3) wenigstens eine 2x2-Matrix von lichtempfindlichen Sensorelementen (3a, 3b) umfasst, die abwechselnd einen Grünfilter und keinen Filter aufweisen.

3. Verfahren nach einem der vorhergehenden Ansprüche,
**dadurch gekennzeichnet, dass**
zur Bestimmung des Grün-Anteils eines Einzelbildes des Kopfes (2a) des Fahrzeugführers (2) das HSV-Farbsystem verwendet wird.

4. Verfahren nach einem der vorhergehenden Ansprüche,
**dadurch gekennzeichnet, dass**
als Bilddaten der Grün-Anteile eines Einzelbildes der Grün-Farbton und dessen Sättigungswerte bestimmt werden.

5. Verfahren nach einem der vorhergehenden Ansprüche,
**dadurch gekennzeichnet, dass**
die Grün-Werte aus jedem Einzelbild extrahiert und der Mittelwert dieser Grün-Werte bestimmt und die für jedes Einzelbild ermittelten Mittelwerte für eine vorbestimmte Zeitdauer gespeichert werden.

6. Verfahren nach einem der vorhergehenden Ansprüche,
**dadurch gekennzeichnet, dass**
die gespeicherten Bilddaten mittels Fourier Transformation (FFT) in den Frequenzbereich transformiert werden und aus dessen Energiespektrum die plethysmographische Größe bestimmt wird.

7. Verfahren nach einem der vorhergehenden Ansprüche ,
**dadurch gekennzeichnet, dass**
zur Bildaufnahme des Kopfes (2a) des Fahrzeugführers (2) eine monochrome Videokamera (6) mit einem separaten Grün-Filter (4) verwendet wird.

8. Verfahren nach einem der vorhergehenden Ansprüche,
**dadurch gekennzeichnet, dass**
zur Ausleuchtung des Kopfes (2a) des Fahrzeugführers (2) eine rote oder infrarote Lichtquelle (7) vorgesehen ist, wenn der strukturierte Grünfilter (4) nur grüne Filterpixel (4c) aufweist oder der Bildsensor (3) nur lichtempfindliche Sensorelemente (3b) mit Grünfilter aufweist.

## Claims

1. Method for identifying the state of a vehicle driver (2) of a motor vehicle (1) by producing a plethysmographic variable indicating the physiological state of the vehicle driver (2), preferably the heart rate, by means of a sensor (3) arranged in the vehicle (1), wherein
- the sensor is embodied as a monochrome image sensor (3) for recording an image of the head (2a) of the vehicle driver (2),
- provision is made of a structured green filter (4, 4a), which has green and transparent filter pixels (4b, 4c),
- the facial region of the vehicle driver (2) is detected from the image recording of the head (2a),
- the green components of the facial region are captured from a plurality of individual images of the head (2a) of the vehicle driver (2) and in each case stored as image data over a predetermined time period, and
- the plethysmographic variable is determined from the stored image data by means of a mathematical algorithm,
**characterized in that**
the green filter (4, 4a) comprises at least one 2 x 2 matrix of filter pixels (4b, 4c) which alternately has green filter pixels (4c) and transparent filter pixels (4b).

2. Method for identifying the state of a vehicle driver (2) of a motor vehicle (1) for producing a plethysmographic variable indicating the physiological state of the vehicle driver (2), preferably the heart rate, by means of a sensor (3) arranged in the vehicle (1), wherein
- the sensor is embodied as an image sensor (3) for recording an image of the head (2a) of the vehicle driver (2) and having light-sensitive sensor elements (3a, 3b) arranged in columns and rows, wherein light-sensitive sensor elements (3b) have a green filter in a pattern,
- the facial region of the vehicle driver (2) is detected from the image recording of the head (2a),
- the green components of the facial region are captured from a plurality of individual images of the head (2a) of the vehicle driver (2) and in each case stored as image data over a predetermined time period, and
- the plethysmographic variable is determined from the stored image data by means of a mathematical algorithm,
**characterized in that**
the image sensor (3) comprises at least one 2 x 2 matrix of light-sensitive sensor elements (3a, 3b) which alternately have a green filter and no filter.

3. Method according to any one of the preceding claims,
**characterized in that**
the HSV colour space is used for determining the green component of an individual image of the head (2a) of the vehicle driver (2).

4. Method according to any one of the preceding claims,
**characterized in that**
the green hue and the saturation values thereof are determined as image data of the green components of an individual image.

5. Method according to any one of the preceding claims,
**characterized in that**
the green values are extracted from each individual image and the mean value of these green values is determined and the mean values ascertained for each individual image are stored for a predetermined time period.

6. Method according to any one of the preceding claims,
**characterized in that**
the stored image data are transformed into the frequency domain by means of a Fourier transform (FFT) and the plethysmographic variable is determined from the energy spectrum thereof.

7. Method according to any one of the preceding claims,
**characterized in that**
a monochrome video camera (6) having a separate green filter (4) is used for recording an image of the head (2a) of the vehicle driver (2).

8. Method according to any one of the preceding claims,
**characterized in that**
a red or infrared light source (7) is provided for illuminating the head (2a) of the vehicle driver (2) when the structured green filter (4) only has green filter pixels (4c) or the image sensor (3) only has light-sensitive sensor elements (3b) with a green filter.

## Revendications

1. Procédé de reconnaissance de l'état d'un conducteur de véhicule (2) d'un véhicule automobile (1) en générant une grandeur pléthysmographique indiquant l'état physiologique du conducteur de véhicule (2), de préférence la fréquence cardiaque au moyen d'un capteur (3) disposé dans le véhicule (1),
- le capteur étant réalisé sous la forme d'un capteur d'images (3) monochrome destiné à enregistrer l'image de la tête (2a) du conducteur de véhicule (2),
- un filtre vert structuré (4, 4a) étant présent, lequel possède des pixels de filtre (4b, 4c) verts et transparents,
- la zone du visage du conducteur de véhicule (2) étant détectée à partir de l'image enregistrée de la tête (2a),
- les parts vertes de la zone d'image étant acquises par plusieurs images individuelles de la tête (2a) du conducteur de véhicule (2) et étant respectivement mises en mémoire en tant que données d'images pendant une durée prédéfinie, et
- la grandeur pléthysmographique étant déterminée à partir des données d'image mises en mémoire au moyen d'un algorithme mathématique,
**caractérisé en ce que**
le filtre vert (4, 4a) comporte au moins une matrice 2x2 de pixels de filtre (4b, 4c) qui possède des pixels de filtre verts (4c) et des pixels de filtre transparents (4b) en alternance.

2. Procédé de reconnaissance de l'état d'un conducteur de véhicule (2) d'un véhicule automobile (1) destiné à générer une grandeur pléthysmographique indiquant l'état physiologique du conducteur de véhicule (2), de préférence la fréquence cardiaque au moyen d'un capteur (3) disposé dans le véhicule (1),
- le capteur étant réalisé sous la forme d'un capteur d'images (3) destiné à enregistrer l'image de la tête (2a) du conducteur de véhicule (2) et étant configuré avec des éléments capteurs photosensibles (3a, 3b) disposés en colonnes et en lignes, les éléments capteurs photosensibles (3b) dans un modèle possédant un filtre vert,
- la zone du visage du conducteur de véhicule (2) étant détectée à partir de l'image enregistrée de la tête (2a),
- les parts vertes de la zone d'image étant acquises par plusieurs images individuelles de la tête (2a) du conducteur de véhicule (2) et étant respectivement mises en mémoire en tant que données d'images pendant une durée prédéfinie, et
- la grandeur pléthysmographique étant déterminée à partir des données d'image mises en mémoire au moyen d'un algorithme mathématique,
**caractérisé en ce que**
le capteur d'images (3) comporte au moins une matrice 2x2 d'éléments capteurs photosensibles (3a, 3b) qui possèdent, en alternance, un filtre vert et aucun filtre.

3. Procédé selon l'une des revendications précédentes, **caractérisé en ce que** le système de couleurs HSV est utilisé pour déterminer la part verte d'une image individuelle de la tête (2a) du conducteur de véhicule (2).

4. Procédé selon l'une des revendications précédentes, **caractérisé en ce que** les données d'image déterminées sont la nuance verte et ses valeurs de saturation des parts vertes d'une image individuelle.

5. Procédé selon l'une des revendications précédentes, **caractérisé en ce que** les valeurs du vert sont extraites de chaque image individuelle et la valeur moyenne de ces valeurs du vert sont déterminées, puis les valeurs moyennes déterminées pour chaque image individuelle sont mises en mémoire pendant une durée prédéfinie.

6. Procédé selon l'une des revendications précédentes, **caractérisé en ce que** les données d'image mises en mémoire sont transformées dans la plage de fréquences au moyen d'une transformation de Fourier (FFT) et la grandeur pléthysmographique est déterminée à partir de son spectre d'énergie.

7. Procédé selon l'une des revendications précédentes, **caractérisé en ce qu'**une caméra vidéo (6) comprenant un filtre vert (4) séparé est utilisée pour l'enregistrement de l'image de la tête (2a) du conducteur de véhicule (2).

8. Procédé selon l'une des revendications précédentes, **caractérisé en ce qu'**une source de lumière (7) rouge ou infrarouge est présente pour l'éclairage de la tête (2a) du conducteur de véhicule (2) lorsque le filtre vert structuré (4) ne possède que des pixels de filtre (4c) verts ou le capteur d'images (3) ne possède que des éléments capteurs (3b) avec filtre vert.
